# EUROPÄISCHE PATENTSCHRIFT

(11) **EP 0 983 021 B1**
(45) Veröffentlichungstag und Bekanntmachung des Hinweises auf die Patenterteilung: **23.08.2006**
(21) Anmeldenummer: 98925872.8
(22) Anmeldetag: 07.05.1998
(51) Int. Cl.: A61B 10/00

(54) **Endoskop für die Feinnadelaspriationsbiopsie**
Endoscope for fine needle aspiration biopsy
Endoscope pour biopsie par aspiration à l'aiguille fine

(30) Priorität: 07.05.1997 DE 29708149 U
(43) Veröffentlichungstag der Anmeldung: 08.03.2000
(73) Patentinhaber: Binmoeller, Kenneth F., San Diego, CA 92121 (US)
(72) Erfinder: Binmoeller, Kenneth F., San Diego, CA 92121 (US)
(74) Vertreter: König, Reimar
(86) Internationale Anmeldenummer: PCT/IB1998/000942
(87) Internationale Veröffentlichungsnummer: WO 1998/049943

(56) Entgegenhaltungen:
- DE-A- 1 791 178
- DE-A- 3 844 131
- DE-A- 4 419 894
- US-A- 2 725 878
- US-A- 4 578 061
- US-A- 4 763 667
- US-A- 5 234 426
- US-A- 5 342 296
- US-A- 5 342 394

## Beschreibung

Die Erfindung betrifft eine Vorrichtung zur endoskopischen Feinnadelbiopsie.

Zur Entnahme einer Gewebeprobe ist es in der Medizin bekannt, Gewebe mit einer Hohlnadel zu punktieren und so biologisches Material für histologische, zytologische oder gentechnologische Untersuchungen zu gewinnen.

Speziell für die zytologische Untersuchung ist die Feinnadelbiopsie, bei der Zellmaterial durch Aspiration mit Hilfe einer dünnen Hohlnadel entnommen wird, bekannt. Die Gewebeentnahme und Positionierung der Hohlnadel wird üblicherweise mit Ultraschall überwacht. Die Überwachung durch Ultraschall ist insbesondere dann erforderlich, wenn Gewebe aus einem klar definierten Bereich entnommen werden soll und dieser Bereich nur eine geringe Ausdehnung hat.

Bei der perkutanen Hohlnadelbiopsie wird die Nadel durch die Haut des Patienten bis zu dem Gewebe vorgeschoben, von dem eine Gewebeprobe entnommen werden soll. Die perkutane Hohlnadelbiopsie eignet sich für die Entnahme von Gewebe, das über die Haut direkt problemlos zu erreichen ist. Gebräuchliche Nadeln besitzen eine Länge von 6 bis 10 cm und werden mit Hilfe eines bildgebenden Verfahrens, wie Ultraschall oder Computertomographie überwacht. Die Ausbeute der Gewebeprobe sowie das Penetrieren verhärteten Gewebes kann bei der verhältnismäßig geringen Nadellänge durch ruckartiges Vorantreiben der Nadel verbessert werden. Dieser Vorgang kann auch durch Verwendung von Federkraft unterstützt werden.

Bestimmte Gewebe lassen sich mit einer perkutanen Biopsienadel nicht punktieren, da das Gewebe nicht erreichbar ist. So kann anderes Gewebe den Weg der Biopsienadel versperren.

Hierzu ist es bekannt, die Biopsienadel durch ein Echoendoskop zu führen und die Gewebeentnahme über ein Hohlorgan durchzuführen. Das Echoendoskop besitzt dabei nicht nur die Funktion einer optischen Überwachung der Punktion, sondern ist gleichzeitig Vehikel und Schutzmantel zur Überwindung großer Wegstrecken und Positionierung der Nadel am Entnahmeort.

Eine endoskopische Punktionsnadelvorrichtung ist aus dem Dokument DE-A- 44 19 894 bekannt.

Die Technik der echoendoskopischen Feinnadelbiopsie ist beispielsweise einsetzbar bei der Entnahme von Bauchspeicheldrüsen- oder Lymphknotengewebe. Das für diese Technik geeignete Echoendoskop besitzt einen Kanal für die Biopsienadel. Die Nadel wird mit dem Echoendoskop in das Hohlorgan eingeführt und in die Nähe der Entnahmestelle gebracht. An der Entnahmestelle wird die Nadel in das Gewebe eingeführt und durch Hin- und Herbewegen mit Hilfe von Unterdruck eine Probe aus dem Gewebe gelöst. Dabei ist ein mehrfaches Hin- und Herbewegen der Nadel erforderlich, um ausreichendes Material für eine zytologische oder histologische Untersuchung zu erhalten.

Schwierigkeiten ergeben sich bei dieser Technik bereits aus der erheblichen Nadellänge, die für einen Zugriff auf das zu entnehmende Gewebe über ein Hohlorgan erforderlich ist. Die Nadellänge muß dabei größer als die Länge des Endoskops sein. So müssen Gewebe mit Nadeln von einer Länge von etwa 160 cm entnommen werden, mehr als das Zehnfache der Nadellänge von Nadeln für die perkutane Biopsie.

Die Bedienung des Endoskops verlangt bereits ein gewisses Geschick. Bei der Positionierung des Endoskops ist die Nadel, die im Bereich des Griffs des Echoendoskops bedient wird, schwer zu kontrollieren. Dies insbesondere, wenn über den Nadel- bzw. Endoskopverlauf mehrere Kurven zu überbrücken sind. Die für den Vorschub der Nadel erforderliche Kraft nimmt mit jeder Kurve aufgrund der auftretenden Reibungskräfte zu. Die Summe der Reibungskräfte kann erheblich sein und muß dann von Hand durch den Anwender überwunden werden. Dies erschwert nicht nur ein genaues Arbeiten und birgt bei sehr feinen Nadeln die Gefahr eines Abknickens der Nadel in sich, sondern kann im Einzelfall auch zur Beschädigung des empfindlichen Echoendoskops führen.

Die echoendosonographiegebundene Feinnadelbiopsie scheitert gänzlich, wenn das zu entnehmende Gewebe eine gewisse Härte aufweist, wie es beispielsweise bei pathologisch verhärtetem Gewebe der Fall ist. In diesem Fall läßt sich die Nadel nicht in das Gewebe einführen, da der auf die Nadel ausgeübte Druck zu groß wird und im besten Falle zu einem Ausweichen des Nadelschaftes bzw. des mit der Nadel verbundenen Endoskops führt, aber auch eine Beschädigung des Endoskops zur Folge haben kann.

Selbst bei leicht penetrierbarem Gewebe ist die Gewebeausbeute häufig nicht zufriedenstellend. Dies ist insbesondere von Nachteil, wenn das punktierte Gewebe für eine zytologische oder histologische Untersuchung vorgesehen ist. Die Biopsienadel muß dann, zur Erhöhung der Ausbeute mehrfach hin- und herbewegt werden. Aber auch diese Technik liefert häufig keine brauchbare Gewebeprobe.

Der Erfindung liegt daher das Problem zugrunde, eine verbesserte endoskopische Biopsievorrichtung zu schaffen, die sich außerdem für den Einsatz bei verhärtetem oder hartem Gewebe eignet.

Die Lösung des Problems basiert auf dem Gedanken, die Bedienung der Nadel zu automatisieren und die Nadel beispielsweise durch eine Feder vorzuspannen. Beim Lösen der Feder schnellt die Nadel in Richtung des Gewebes nach vorne. Aufgrund der Trägheit der Masse des Echoendoskops durchdringt die Nadel auch verhärtetes Gewebe. Durch die hohe Beschleunigung der Nadel wird die Gefahr eines Abknickens verringert und Reibungskräfte überwunden.

Das erfindungsgemäße Problem wird gelöst durch eine Vorrichtung gemäß Anspruch 1, bei der die vorgespannte Nadel nach Betätigen eines Auslösers an einer Vorspanneinrichtung automatisch vorschnellt.

Mit der erfindungsgemäß automatisierten Biopsienadel lassen sich hochwertige Gewebeproben erzeugen und selbst verhärtetes Gewebe durchdringen, bei dem mit der herkömmlichen manuellen Biopsienadel eine Gewebeentnahme über das Endoskop nicht mehr möglich ist oder der Versuch zur Beschädigung der Nadel bzw. des Endoskops führt.

Zusätzlich führt der Einsatz der erfindungsgemäßen Biopsienadel zu einer erheblichen Erhöhung der Gewebeausbeute, wobei das unerwünschte mehrfache hin- und herbewegen der Nadel vermieden wird.

Dabei kann die Nadel im Kanal des Echoendoskops von einem Schutzmantel umgeben sein, der vorzugsweise aus einer Spiralscheide besteht, die sich unabhängig von der Nadel verschieben läßt. Mit Hilfe des verschiebbaren Mantels kann die Nadel auch nach dem Austreten aus dem Kanal des Echoendoskops begleitet bzw. verstärkt und besser positioniert werden.

Zur Sicherung des Vortriebs der Biopsienadel und Vermeidung eines Herausspringens der Vorspanneinrichtung kann deren Gehäuse in die Kanalöffnung des Echoendoskops eingeschraubt sein. Durch entsprechende Justiermittel kann dabei eine genaue Einstellung der Nadelposition und des Vortriebs an der Vorspanneinrichtung vorgenommen werden.

In einer besonderen Ausführungsform erlaubt die erfindungsgemäße Vorrichtung neben der automatischen Gewebeentnahme die herkömmliche Funktion einer manuellen Entnahme durch Hin- und Herbewegen der Biopsienadel. So kann vor oder sogar während der Gewebeentnahme entschieden werden, ob die Gewebeprobe auf herkömmliche Weise durch Hin- und Herschieben der Nadel (manuell) oder mit Hilfe der vorschnellenden Nadel (automatisiert) entnommen wird.

Im folgenden wir die Erfindung anhand eines in der Zeichnung dargestellten Ausführungsbeispiels des näheren erläutert.

In der Zeichnung zeigen:

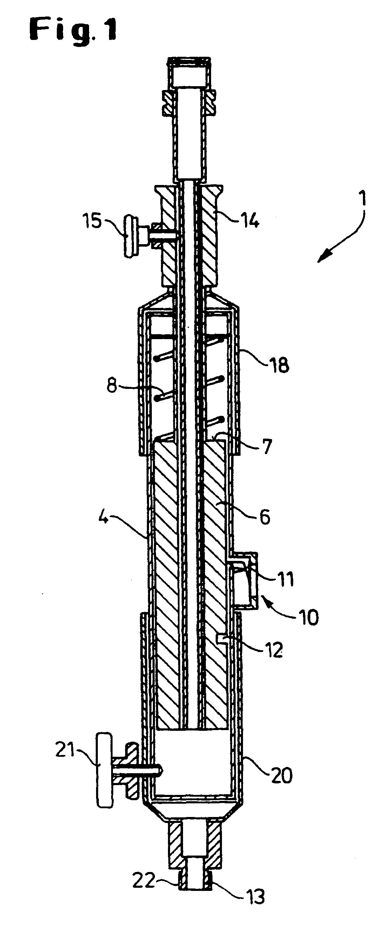

- Fig. 1: eine schematische Darstellung der Vorspanneinrichtung des Biopsiegerätes;

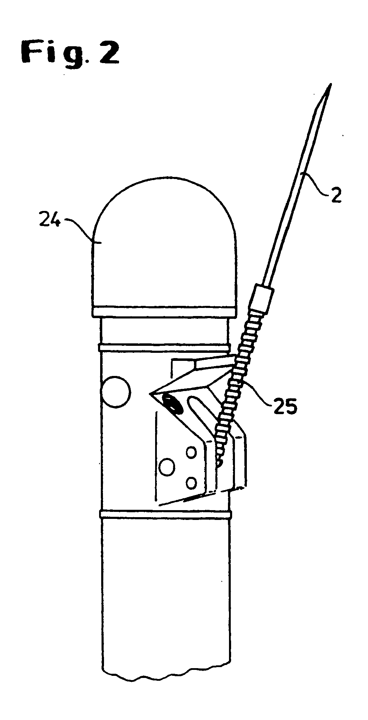

- Fig. 2: eine Vorspanneinrichtung am Bedienungsende des Echoendoskops;

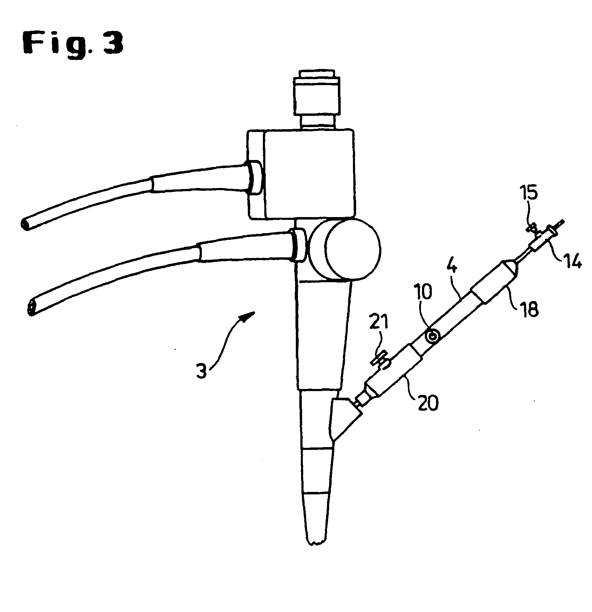

- Fig. 3: eine aus dem Untersuchungsende des Echoendoskops heraustretende Biopsienadel.

Die Biopsienadel besteht aus einer Bedien- und Vorspanneinrichtung 1 mit einem Hauptzylinder 4, in dem sich ein Gleitkolben 6 befindet. Der Gleitkolben 6 besitzt an seiner Oberseite einen Vorsprung 7, an dem eine Feder 8 zum Vorspannen der Nadel 2 anliegt. An dem Hauptzylinder befindet sich ein Auslöser 10 mit einer Feder 11, die in eine Nut 12 des Gleitkolbens 6 eingreift, wenn die Biopsienadel 2 bzw. der Gleitkolben 6 gespannt ist. Am nadelfernen Ende des Gleitkolbens befindet sich ein Griff 14, mit dem der Kolben 6 gespannt wird, mit dem aber auch eine manuelle Biopsie durchgeführt werden kann. An dem Griff befindet sich eine Arretierschraube 15, mit der die Biopsienadel 2 fixiert wird. In entspanntem Zustand der Feder 8 liegt der Griff 14 an einer Kalibrierhülse 18 an. Mit Hilfe der Kalibrierhülse 18 läßt sich eine Feineinstellung der Endposition des Kolbens 6 und damit der Vortriebstiefe der Biopsienadel 2 vornehmen.

Auf dem nadelseitigen Ende des Hauptzylinders 4 befindet sich eine Buchse 20, mit deren Ende die Vorspann- und Kontrolleinrichtung in dem endoskopseitigen Nadelkanal fixiert wird. Mit einer Arretierschraube 21 ist der Hauptzylinder 4 in der Buchse 20 fixiert. Die Buchse 20 wird mit Hilfe eines Schraubverschlusses 22 in der Nadelkanalöffnung des Echoendoskops 3 fixiert. Als Echoendoskop kann ein Olympus GF-UM 30P TM der Firma Olympus Optical Company, Tokyo, Japan verwendet werden. Dieses Echoendoskop besitzt einen Nadelkanal mit 2,8 mm Durchmesser mit einer Austrittsöffnung in direkter Nähe der Fiberglasoptik. Der Austrittswinkel der Nadel läßt sich am Echoendoskop verstellen. Der am Ende des Echoendoskops befindliche Meßwertaufnehmer 24 ist von einem Latex-Ballon umgeben. Dieser kann beim Einsatz des Echoendoskops mit Wasser gefüllt werden, welches dann als Medium zwischen dem Meßwertaufnehmer 24 und beispielsweise der Darmwand dienen kann.

Die Biopsienadel 2 befindet sich in einer Führungshülse und wird über die Bedien- und Vorspanneinrichtung 1 in das Echoendoskop eingeführt, bis die Nadel mit der Hülse am unteren Ende des Echoendoskops austritt. Dabei ist es vorteilhaft, wenn die Nadelspitze abgeschrägt und das distale Ende der Nadel 2 zur Verbesserung der Auflösung im Ultraschall sandgestrahlt ist.

In der Hohlnadel befindet sich ein stumpfes Stilett, welches 2 mm aus dem offenen Nadelende herausragt.

Die in die Bedien- und Vorspanneinrichtung 1 eingeführte Biopsienadel 2 ist über ihre Schutzhülse mit dem Griff 14 der Bedien- und Vorspanneinrichtung 1 verschraubt.

Für die Punktion kann die Nadel über den Griff 14 vor und zurückbewegt werden. In dieser Weise läßt sich die erfindungsgemäße Vorrichtung in herkömmlicher Weise manuell einsetzen.

Durch übermäßiges Zurückziehen des Gleitkolbens 6 bewegt sich die Nut 12 zur Feder 11, wobei sich die Spiralfeder 8 auflädt. Beim Einrasten der Feder 11 in die Nut 12 ist die Biopsienadel 2 vorgespannt und kann über den Auslöser 10 schlagartig vorwärtsbewegt werden. Über die Kalibrierhülse 18 läßt sich die Eindringtiefe der Biopsienadel 2 voreinstellen. Eine Grobeinstellung ist über die Einschubtiefe des Hauptzylinders 4 in die Hülse 20 möglich, wobei der Hauptzylinder 4 über die Schraube 21 fixiert wird.

Mit Hilfe der an den jeweiligen Enden des Hauptzylinders 4 befindliche Hülse 20 läßt sich eine schnelle und akkurate Einstellung der Biopsienadel vornehmen. Dafür wird die im Gleitkolben 6 des Hauptzylinders 4 befindliche Biopsienadel 2 bei gelöster Arretierschraube 21 in die Hülse 20 eingeschoben, bis die Nadel 2 im Echoendoskop sichtbar wird. Nun wird die Arretierschraube 21 angezogen und die genaue Eindringtiefe über die Kalibrierhülse 18 eingestellt. Durch geringfügiges Herausziehen des Stiletts aus der Hohlnadel 2 wird das scharfe Ende der Hohlnadel freigegeben, um zunächst Gewebe wie z.B. die Darmwand zu penetrieren und unter sonographischer Kontrolle in die Nähe des zu punktierenden Gewebes zu gelangen.

Sobald die Nadel das zu punktierende Gewebe erreicht hat, wird das Stilett entfernt und beispielsweise eine Spritze zum Erzeugen eines Unterdrucks am anderen Ende der Hohlnadel 2 aufgesetzt.

Mit Hilfe des Griffs 14 kann nun die Nadel in das zu punktierende Gewebe eingeführt werden. Zur manuellen Punktierung wird die Arretierschraube 21 gelöst und der Hauptzylinder hin- und herbewegt. Bereitet die manuelle Punktion Schwierigkeiten oder läßt sich das Gewebe nicht ohne weiteres penetrieren, wird die Sprungfeder über den Auslöser 10 ausgelöst, wobei die Nadel in das verhärtete Gewebe vorschnellt.

## Patentansprüche

1. Endoskop für die Feinnadelaspirationsbiopsie mit einem Nadelkanal und einer das Endoskop durchlaufenden Hohlnadel (2), **dadurch gekennzeichnet, dass** die Hohlnadel mit einer Einrichtung zum Vorspannen (1) und Auslösen (10) der vorgespannten Nadel (2) verbunden ist.

2. Endoskop nach Anspruch 1, **dadurch gekennzeichnet, dass** die Vorspanneinrichtung in einem Gehäuse (4, 18, 20) angeordnet ist, das in die Kanalöffnung am Bedienkopf des Endoskops eingeschraubt ist.

3. Endoskop nach einem der Ansprüche 1 oder 2, **dadurch gekennzeichnet, dass** die Vorspanneinrichtung (1) eine Buchse (20) zur Positionierung der Nadel (2) besitzt.

4. Endoskop nach einem der Ansprüche 1 bis 3, **dadurch gekennzeichnet, dass** die Vorspanneinrichtung (1) eine Kalibrierhülse (18) besitzt, mit der die Feineinstellung der Vortriebstiefe der Biopsienadel (2) möglich ist.

5. Endoskop nach einem oder mehreren der Ansprüche 1 bis 4, **dadurch gekennzeichnet, dass** die Vorspanneinrichtung einen Federmechanismus (6, 8) enthält und daß sich die Nadel (2) von dem Federmechanismus (6, 8) entkoppeln lässt, um eine manuelle Nadelführung zu ermöglichen.

6. Endoskop nach einem oder mehreren der Ansprüche 1 bis 5, **dadurch gekennzeichnet, dass** die Biopsienadel (2) im Bereich ihrer Spitze sandgestrahlt ist.

7. Endoskop nach einem oder mehreren der Ansprüche 1 bis 6, **dadurch gekennzeichnet, dass** die Nadel (2) mit Ausnahme der Nadelspitze von einer Spiralscheide (25) umgeben ist.

8. Endoskop nach Anspruch 7, **dadurch gekennzeichnet, dass** die Spiralscheide (25) sich unabhängig von der Nadel im Kanal des Endoskops verschieben lässt.

9. Endoskop nach einem oder mehreren der Ansprüche 1 bis 8, **gekennzeichnet durch** einen mit einem Medium füllbaren Latexballon im Bereich des Messwertaufnehmers (24) des Endoskops, wobei das Endoskop ein Echoendoskop ist.

10. Endoskop nach einem oder mehreren der Ansprüche 1 bis 9, **dadurch gekennzeichnet, dass** die Vorspanneinrichtung (1) am Instrumentenkanal des Endoskops befestigt ist.

## Claims

1. Endoscope for fine needle aspiration biopsy with a needle channel and a hollow needle (2) running through the endoscope, **characterised in that** the hollow needle is connected to a device for pre-tensioning (1) and releasing (10) the pre-tension needle (2).

2. Endoscope according to claim 1, **characterised in that** the pre-tension device is arranged in a housing (4, 18, 20), which is screwed into the channel opening at the operating head of the endoscope.

3. Endoscope according to either of claims 1 or 2, **characterised in that** the pre-tension device (1) has a bush (20) for positioning of the needle (2).

4. Endoscope according to any one of claims 1 to 3, **characterised in that** the pre-tension device (1) has a calibration sleeve (18) with which fine adjustment of the penetration depth of the biopsy needle (2) is possible.

5. Endoscope according to one or more of claims 1 to 4, **characterised in that** the pre-tension device contains a spring mechanism (6, 8) and **in that** the needle (2) can be de-coupled from the spring mechanism (6, 8) in order to allow manual guidance of the needle.

6. Endoscope according to one or more of claims 1 to 5, **characterised in that** the biopsy needle (2) is sand-blasted in the area of its tip.

7. Endoscope according to one or more of claims 1 to 6, **characterised in that** the needle (2) with the exception of the needle tip is surrounded by a spiral sheath (25).

8. Endoscope according to claim 7, **characterised in that** the spiral sheath (25) can be moved independently of the needle in the channel of the endoscope.

9. Endoscope according to one or more of claims 1 to 8, **characterised by** a latex balloon which can be filled with a medium in the area of the measurement value sensor (24) of the endoscope, the endoscope being an echoendoscope.

10. Endoscope according to one or more of claims 1 to 9, **characterised in that** the pre-tension device (1) is attached to the instrument channel of the endoscope.

## Revendications

1. Endoscope pour la biopsie par aspiration via une aiguille fine, comprenant un canal d'aiguille et une aiguille creuse (2) qui traverse l'endoscope, **caractérisé en ce que** l'aiguille creuse est reliée à un dispositif pour mettre l'aiguille sous tension (1) et pour déclencher (10) l'aiguille (2) sous tension.

2. Endoscope selon la revendication 1, **caractérisé en ce que** le dispositif de mise sous tension est agencé dans un boîtier (4, 18, 20) qui est vissé dans l'ouverture du canal au niveau de la tête de manipulation de l'endoscope.

3. Endoscope selon l'une des revendications 1 ou 2, **caractérisé en ce que** le dispositif de mise sous tension (1) possède une douille (20) pour le positionnement de l'aiguille (2).

4. Endoscope selon l'une des revendications 1 à 3, **caractérisé en ce que** le dispositif de mise sous tension (1) possède une douille de calibrage avec laquelle il est possible d'effectuer un réglage fin de la profondeur d'enfoncement de l'aiguille de biopsie (2).

5. Endoscope selon l'une ou plusieurs des revendications 1 à 4, **caractérisé en ce que** le dispositif de mise sous tension comprend un mécanisme à ressort (6, 8), et **en ce que** l'aiguille (2) peut être découplée du mécanisme à ressort (6, 8) pour permettre un guidage manuel de l'aiguille.

6. Endoscope selon l'une ou plusieurs des revendications 1 à 5, **caractérisé en ce que** l'aiguille de biopsie (2) est sablée dans la région de sa pointe.

7. Endoscope selon l'une ou plusieurs des revendications 1 à 6, **caractérisé en ce que** l'aiguille (2) est entourée par une gaine spiralée (25), à l'exception de la pointe de l'aiguille.

8. Endoscope selon la revendication 7, **caractérisé en ce que** la gaine spiralée (25) peut être déplacée dans le canal de l'endoscope indépendamment de l'aiguille.

9. Endoscope selon l'une ou plusieurs des revendications 1 à 8, **caractérisé par** un ballon en latex susceptible d'être rempli avec un fluide, dans la région du capteur de mesure (24) de l'endoscope, de sorte que l'endoscope est un échoendoscope.

10. Endoscope selon l'une ou plusieurs des revendications 1 à 9, **caractérisé en ce que** le dispositif de mise sous tension (1) est fixé dans le canal à instrument de l'endoscope.
